**Europäisches Patentamt**

**European Patent Office**

**Office européen des brevets**

(11) Numéro de publication : **0 365 403 B1**

(12) ## FASCICULE DE BREVET EUROPEEN

(45) Date de publication du fascicule du brevet :
28.10.92 Bulletin 92/44

(51) Int. Cl.⁵ : **C07C 31/38, C07C 29/136**

(21) Numéro de dépôt : **89402826.5**

(22) Date de dépôt : **13.10.89**

(54) **Procédé de préparation de trifluoroethanol.**

(30) Priorité : **21.10.88 FR 8813797**

(43) Date de publication de la demande :
**25.04.90 Bulletin 90/17**

(45) Mention de la délivrance du brevet :
**28.10.92 Bulletin 92/44**

(84) Etats contractants désignés :
**AT BE CH DE ES FR GB GR IT LI LU NL SE**

(56) Documents cités :
**EP-A- 0 125 951**
**EP-A- 0 128 059**
**EP-A- 0 177 393**

(73) Titulaire : **RHONE-POULENC CHIMIE**
**25, quai Paul Doumer**
**F-92408 Courbevoie Cédex (FR)**

(72) Inventeur : **Cordier, Georges**
**domicilié 50 Chemin des Herminières**
**F-69340 Francheville (FR)**

(74) Mandataire : **Fabre, Madeleine-France et al**
**RHONE-POULENC INTERSERVICES Service**
**Brevets Chimie 25, quai Paul Doumer**
**F-92408 Courbevoie Cédex (FR)**

## Description

La présente invention concerne un procédé de préparation de trifluoroéthanol. Elle concerne plus particulièrement un procédé de préparation de trifluoroéthanol par hydrogénation de l'acide trifluoroacétique.

Il est connu de préparer les alcools perfluoroalkylés à partir des acides correspondant par au moins deux techniques. La première en phase vapeur, la deuxième en phase liquide.

Selon la première technique on met en contact tel que décrit par exemple dans les brevets US 3390191 et US 4273947 l'acide carboxylique perfluoroalkylé sous forme gazeuse avec un catalyseur à base de chrome ou de cuivre pour le brevet US 3390191 ou à base de rhodium ou d'irridium par le brevet US 4273947 à une température comprise entre le point d'ébullition de l'acide et 400°C. Les deux brevets décrivent uniquement l'hydrogénation de l'acide trifluoroacétique et les rendements annoncés sont souvent faibles 1,4% par le brevet US 4273947 et 37% par le brevet US 3390191. Il est aussi connu un procédé continu d'hydrogénation de l'acide trifluoroacétique tel que décrit dans le brevet US 4396784 qui consiste à hydrogéner cet acide sur un catalyseur à base de rhénium déposé selon un rapport pondéral de 3,3% sur une alumine fluorée. Le procédé est réalisé à une température de 280°C sous une pression de 20 bars.

Les faibles rendements, les températures nécessaires pour obtenir ces rendements, la technologie nécessaire à la mise en oeuvre de réactions en phase gazeuse font que l'industrie chimique préfère utiliser des procédés en phase liquide.

La deuxième technique de préparation du trifluoroethanol en phase liquide a été notamment décrite dans les brevets US 3663629 et US 4273947. Selon ces brevets on met en contact dans un autoclave l'acide perfluoroalkylcarboxylique, l'hydrogène avec un catalyseur choisi parmi le ruthenium pour le brevet US 3663629 et le rhodium ou l'irridium par le brevet US 4273947. La pression d'hydrogène utilisée est très élevée dans le brevet US 3663629 puisqu'elle est de préférence comprise entre 40 et 400 bars. Pour le second elle est beaucoup plus faible puisqu'elle est comprise entre 5 et 15 bars. Par contre, la quantité volumique d'acide introduit calculée par rapport au volume total du réacteur qui est de un pour cent ne permet pas d'envisager de production industrielle. L'ensemble des procédés d'hydrogénation par une technique en phase liquide est toujours difficile à extrapoler à l'échelle industrielle car ce sont toujours des procédés discontinus qui imposent des temps de vidange et nettoyage des réacteurs qui ont des conséquences néfastes sur le prix de revient du produit final.

La demande de brevet européen publiée sous le N° 0 128 059 concerne la synthèse de fabrication des perfluoroalcanols à partir d'acides perfluorocarboxyliques et vise l'amélioration apportée par un additif choisi parmi les ions halogénures et divers cations métalliques mais elle ne traite pas de passages à l'échelle industrielle.

La demande de brevet européen publiée sous le N° 0125 951 vise un procédé du même type mais mené en discontinu étendait à résoudre les problèmes du discontinu.

L'ensemble de ces documents prouve que le problème que cherche à résoudre l'industrie, avoir un procédé de préparation de trifluoroethanol à partir de l'acide trifluoroacétique en phase liquide, en continu, sur un catalyseur aisément disponible et avec d'excellents rendements n'est pas évident.

La présente invention a néanmoins permis d'atteindre cet objectif. Elle a pour objet un procédé de préparation de trifluoroéthanol par hydrogénation de l'acide trifluoroacétique en présence d'un catalyseur à base de rhodium ou de ruthenium caractérisé en ce qu'on introduit en continu selon un mode ruisselant l'acide trifluoroacétique en solution dans un mélange eau/trifluoroéthanol sur un lit fixe constitué par le métal catalyseur déposé sur un support selon une concentration en poids comprise entre 0,5 et 5 % et de préférence entre 0,5 et 2%, par rapport au support, en présence d'hydrogène et à une température comprise entre 60 et 150°C sous une pression inférieure à 50 bars et de préférence comprise entre 15 et 50 bars.

La solution introduite en continu sur le lit fixe a de préférence la composition suivante

| | |
|---|---|
| acide trifluoroacétique | 5 à 50 parts |
| trifluoroéthanol | 25 à 47,5 parts |
| eau | 25 à 47,5 parts |

Les parts étant fixées en moles.

On préfère tout particulièrement mettre en oeuvre une solution contenant l'acide trifluoroacétique dilué dans un mélange equimolaire eau /trifluoroéthanol.

Le catalyseur à base de rhodium ou de ruthenium est déposé sur un support constitué de silice, de charbon ou d'alumine.

On préfère utiliser un catalyseur à base de rhodium déposé sur charbon.

La quantité de métal catalytique déposée sur le support est comprise de préférence en poids entre 0,5 et 2%.

La granulométrie du support est de préférence comprise entre 1 et 5 mm et tout particulièrement entre 2 et 4 mm. Ce support peut être sous formes diverses : sphères, bâtonnets, brisures ou écailles.

L'hydrogène est introduit pur ou en mélange avec un gaz inerte tel que l'azote, le méthane, dans les conditions de la réaction. Le réacteur est constitué d'un ou plusieurs tubes cylindriques ayant un diamètre compris entre 3 et 40 cm. La hauteur du tube industriel varie avantageusement entre 2 et 10 mètres et de préférence entre 4 et 7 mètres. Le réacteur est de préférence muni d'une enveloppe contenant un fluide caloporteur. Il est alimenté par le fluide liquide constitué du mélange réactionnel à un débit tel qu'en fonction du diamètre du tube la vitesse linéaire de la phase liquide considérée par rapport au diamètre du tube vide soit comprise entre 0,2 et 10 mm s$^{-1}$ et de préférence entre 1 et 5 mm s$^{-1}$.

Le débit d'hydrogène ou du gaz vecteur contenant l'hydrogène est déterminé par la formule :

$$Q_r = (Q_2 \times P_{bar}) \times 1,4$$

dans laquelle $Q_2$ est le débit de liquide en litres/heure et P la pression appliquée dans le tube en bars.

$Q_r$, exprimé en litres/heures signifie le débit de gaz TPN (température et pression normales).

Selon un meilleur procédé de mise en oeuvre le liquide et le gaz sont introduits à co-courant dans les tubes. Ils peuvent aussi sans sortir du cadre de la présente invention être introduits à contre-courant. Pour éviter les hétérogénéités de température dans l'ensemble du lit catalytique il est préférable de chauffer préalablement les réactifs à une température d'environ 10°C inférieure à la température d'hydrogénation.

Le liquide et le gaz effluents de la colonne sont refroidis et séparés notamment par leur différence de point d'ébullition. Dans le cas où la transformation est incomplète et qu'il subsiste encore de l'acide trifluoroacétique un second passage sur le lit catalytique est conseillé.

L'hydrogénation dans les conditions de la présente invention ne produit presque pas de produits secondaires (moins de 0,5%).

L'invention sera plus complètement décrite à l'aide des exemples suivants qui ne doivent pas être considérés comme limitatifs de l'invention.

EXEMPLE 1

Le réacteur est constitué par un tube en Hastelloy "C" matériau résistant à l'acide trifluoroacétique chaud. Il est conçu pour résister à des pressions supérieures ou égales à 150b. Son diamètre intérieur est de 2,6 cm et sa hauteur est de 100 cm.

Il est rempli par 0,5 litre de catalyseur soit un poids, selon la densité de ce dernier de 185 à 195 g. Le catalyseur préféré est un catalyseur contenant 1% de rhodium p/p déposé sur un charbon de grande surface spécifique ( 1 000 m$^2$g$^{-1}$) et présenté en grains de 3 à 4 mm de diamètre et de 3 à 5 mm de longueur.

L'acide trifluoroacétique et l'hydrogène sont alimentés à co-courant de haut en bas en quantité contrôlée.

Dans le réacteur décrit précédemment et rempli avec le catalyseur 1% Rh/C (196 g) on effectue la succession d'opérations suivantes :

Purge à l'azote 20 l/h (1 h) puis purge avec de l'hydrogène 20 l/h (1 h) à la température ambiante.

Injection :

Eau distillée à 90°C/20b d'hydrogène : 8 heures à raison de 1 l h$^{-1}$.

PUIS :

Injection d'un mélange ayant la composition suivante : (mole/mole)

| Acide trifluoroacétique | 25 % |
| Eau | 37,5% |
| Trifluoroethanol | 37,5% |

Cette composition n'est pas critique et on peut injecter de l'acide trifluoroacétique pur ou largement dilué dans de l'eau.

La température du lit est portée à 120°C et la pression totale à 40 bars.

Le débit de mélange liquide est amené à 2 l/h. Le débit d'hydrogène est porté à 110 l h$^{-1}$ TPN. Le mélange liquide effluent recueilli en bas du lit est analysé par acidimétrie et chromatographie phase gazeuse, puis recyclé. Le mélange gazeux effluent est également analysé.

On dose en chromatographie phase gazeuse les produits tels que $CF_3CH_3$, $C_2H_6$, $CH_4$ résultant de l'hydrogénolyse des molécules oxygénées.

EP 0 365 403 B1

RESULTATS

| NOMBRE DE PASSAGE SUR LE LIT CATALYTIQUE | TFE/TFA+TFE mole/mole | TT% TFA | ACTIVITE MOLES TFE FORMEES $h^{-1}$ $Kg^{-1}$ cata |
|---|---|---|---|
| 1 | 0,78 | 45 | 21,5 |
| 2 | 0,9 | 75 | 14,9 |
| 3 | 0,96 | 90 | 7,5 |
| 4 | 0,993 | 98,3 | 3,0 |

Le rendement de la transformation secondaire en la somme ($CH_4$ + $C_2 H_6$ + $CF_3 CH_3$) est constamment compris entre 0,3 et 0,5% de l'acide trifluoroacétique engagé ce qui confère à cette réaction une sélectivité $\geq$ 99,5% pour la formation du trifluoroéthanol.

EXEMPLE N°2

On répète l'exemple 1 à 90°C sous 40 bars de pression totale. Au bout de 8 passages sur le lit catalytique le taux de transformation de l'acide trifluoroacétique est de 98,9% et le rendement en trifluoroéthanol est de 99,9% par rapport à l'acide trifluoroacétique transformé.

EXEMPLE N°3

On répète l'exemple N°1 à 120°C sous 20 bars de pression totale.
Au bout de 8 passages sur le lit catalytique le taux de transformation de l'acide trifluoroacétique est de 99,3% et le rendement en TFE par rapport au TFA engagé est de 99,6%.

**Revendications**

**1.** Procédé de préparation de trifluoroéthanol par hydrogénation de l'acide trifluoroacétique en présence d'un catalyseur à base de rhodium ou de ruthénium caractérisé en ce qu'on introduit en continu selon un mode ruisselant l'acide trifluoroacétique, en solution dans un mélange eau - trifluoroéthanol, sur un lit fixe constitué par le catalyseur déposé sur un support selon une concentration en poids comprise entre 0,5 et 5%, de préférence entre 0,5 et 2% par rapport au support, en présence d'hydrogène et à une température comprise entre 60 et 150°C sous une pression inférieure à 50 bars et de préférence comprise entre 15 et 50 bars.

**2.** Procédé selon la revendication 1 caractérisé en ce que l'acide trifluoroacétique est introduit dans un mélange ayant la constitution suivante en moles.
acide trifluoroacétique      5 à 50 parts
trifluoroéthanol      25 à 47,5 parts
eau      25 à 47,5 parts

**3.** Procédé selon les revendications 1 et 2 caractérisé en ce que l'acide trifluoroacétique est introduit dans un mélange équimoléculaire eau/trifluoroéthanol.

**4.** Procédé selon la revendication 1 caractérisé en ce que le métal est déposé sur un support à base de silice, de charbon ou d'alumine et de préférence à base de charbon.

4

5. Procédé selon la revendication 4 caractérisé en ce que le support a une granulométrie comprise entre 1 et 5 mm et de préférence entre 2 et 4 mm.

6. Procédé selon la revendication 1 caractérisé en ce que le lit catalytique est composé de un ou plusieurs tubes cylindriques ayant un diamètre compris entre 3 et 40 cm et une longueur comprise entre 2 et 10 mètres.

7. Procédé selon la revendication 1 caractérisé en ce que la vitesse de passage de la phase liquide dans le tube catalytique est comprise entre 0,2 et 10 mm s$^{-1}$ et de préférence entre 1 et 5 mm s$^{-1}$.

**Patentansprüche**

1. Verfahren zur Herstellung von Trifluorethanol durch Hydrieren von Trifluoressigsäure in Gegenwart eines Katalysators auf der Basis von Rhodium oder Ruthenium, dadurch gekennzeichnet, daß man kontinuierlich Trifluoressigsäure in Lösung in einem Gemisch aus Wasser und Trifluorethanol auf ein Festbett, das aus dem Katalysator, abgeschieden auf einem Träger in einer Konzentration von 0,5 bis 5 Gew.-%, vorzugsweise von 0,5 bis 2 Gew.-%, bezogen auf den Träger, in Gegenwart von Wasserstoff und bei einer Temperatur im Bereich von 60 bis 150°C unter einem Druck unter 50 bar, vorzugsweise von 15 bis 50 bar, rieseln läßt.

2. Verfahren nach Anspruch 1, dadurch gekennzeichnet, daß die Trifluoressigsäure in Form eines Gemisches eingebracht wird, das folgende Zusammensetzung in mol aufweist:
Trifluoressigsäure        5 bis 50 Teile
Trifluorethanol        25 bis 47,5 Teile
Wasser        25 bis 47,5 Teile.

3. Verfahren nach Anspruch 1 oder 2, dadurch gekennzeichnet, daß die Trifluoressigsäure in einem äquimolaren Gemisch aus Wasser und Trifluorethanol eingebracht wird.

4. Verfahren nach Anspruch 1, dadurch gekennzeichnet, daß das Metall auf einem Träger aus Kieselsäure, Kohlenstoff oder Aluminiumoxid, vorzugsweise Kohlenstoff, abgeschieden ist.

5. Verfahren nach Anspruch 4, dadurch gekennzeichnet, daß der Träger eine Korngrößenverteilung im Bereich von 1 bis 5 mm, vorzugsweise von 2 bis 4 mm, aufweist.

6. Verfahren nach Anspruch 1, dadurch gekennzeichnet, daß das Katalysatorbett aus einem oder mehreren zylindrischen Rohren mit einem Durchmesser von 3 bis 40 cm und einer Länge von 2 bis 10 m besteht.

7. Verfahren nach Anspruch 1, dadurch gekennzeichnet, daß die Rieselgeschwindigkeit der flüssigen Phase in dem Katalysatorrohr 0,2 bis 10 mm.s$^{-1}$, vorzugsweise 1 bis 5 mm.s$^{-1}$ beträgt.

**Claims**

1. Process for the preparation of trifluoroethanol by hydrogenation of trifluoroacetic acid in the presence of a rhodium or ruthenium-based catalyst, characterized in that continuous introduction takes place in a trickling mode of trifluoroacetic acid, dissolved in a water-trifluoroethanol mixture, onto a fixed bed constituted by the catalyst deposited on a support in a concentration by weight between 0.5 and 5%, preferably between 0.5 and 2% based on the support, in the presence of hydrogen and at a temperature between 60 and 150°C and under a pressure below 50 bar and preferably between 15 and 50 bar.

2. Process according to claim 1, characterized in that the trifluoroacetic acid is introduced into a mixture which has a molar constitution of 5 to 50 parts trifluoroacetic acid, 25 to 47.5 parts of trifluoroethanol and 25 to 47.5 parts of water.

3. Process according to claims 1 and 2, characterized in that the trifluoroacetic acid is introduced into an equimolecular water/trifluoroethanol mixture.

4. Process according to claim 1, characterized in that the metal is deposited on a support based on silica, charcoal or alumina and preferably based on charcoal.

5. Process according to claim 4, characterized in that the support has a grain size between 1 and 5 mm and preferably between 2 and 4 mm.

6. Process according to claim 1, characterized in that the catalytic bed is constituted by one or more cylindrical tubes having a diameter between 3 and 40 cm and a length between 2 and 10 m.

7. Process according to claim 1, characterized in that the passage speed of the liquid phase into the catalytic tube is between 0.2 and 10 mm $s^{-1}$ and preferably between 1 and 5 mm $s^{-1}$.